Europäisches Patentamt

European Patent Office

Office européen des brevets

(11) Veröffentlichungsnummer: **0 492 454 A1**

(12) **EUROPÄISCHE PATENTANMELDUNG**

(21) Anmeldenummer: **91121823.8**

(51) Int. Cl.5: **A61M 5/145**

(22) Anmeldetag: **19.12.91**

(30) Priorität: **28.12.90 DE 4042101**

(43) Veröffentlichungstag der Anmeldung:
**01.07.92 Patentblatt 92/27**

(84) Benannte Vertragsstaaten:
**AT BE CH DE DK ES FR GB GR IT LI LU NL SE**

(71) Anmelder: **MEDICAL SUPPORT GMBH**
**Raiffeisenstrasse 2**
**W-6054 Rodgau 2(DE)**

(72) Erfinder: **Der Erfinder hat auf seine Nennung verzichtet**

(74) Vertreter: **Flosdorff, Jürgen, Dr.**
**Alleestrasse 33**
**W-8100 Garmisch-Partenkirchen(DE)**

(54) **Prüfgerät für Spritzen- und Infusionspumpen.**

(57) Das Prüfgerät (1) für Spritzen- und Infusionspumpen enthält eine Glaskolbenspritze (3), ein Linear-Potentiometer (9), dessen Spindel (10) starr mit dem Kolben (7) der Glaskolbenspritze (3) verbunden ist, einen Druck-Meß-Wandler (15), der mit dem Spritzeneinlaß (5) in Verbindung steht, und eine Rechner- und Auswerte-Elektronik (17), die mit dem Linear-Potentiometer (9) verbunden ist. Die zu prüfende Pumpe wird mittels eines wassergefüllten Schlauches mit der Glaskolbenspritze verbunden und drückt mit einer vorgewählten Förderrate Wasser in die Glaskolbenspritze. Dabei zeigt das Gerät auf drei Anzeigefeldern (16,18,19) den Förderdruck, die Förderrate und die Fördermenge an. Mit dem Gerät können diese wichtigen Parameter schnell und präzise gemessen werden.

EP 0 492 454 A1

Die Erfindung betrifft ein Gerät zur Prüfung und Messung der Parameter von Spritzenpumpen und Infusionspumpen, und zwar insbesondere zur Messung der Förderrate, des Förderdrucks, der Fördermenge und des Abschaltdrucks. Diese Parameter sind bei allen Spritzenpumpen und Infusionspumpen in regelmäßigen Abständen zu überprüfen, da von der korrekten Größe dieser Parameter unter Umständen Gesundheit und Leben von Patienten abhängen.

Bisher wird die Förderrate von Spritzenpumpen indirekt über eine Wegmessung des Vorschubs des Spritzenkolbens ermittelt, wozu es bekannt ist, eine mechanische Meßuhr in die Spritzenaufnahmemulde der Spritzenpumpe einzuspannen und die Spritzenpumpe mittels einer Schaltuhr eine bestimmte Zeit lang zu betätigen. Nach Ablauf dieser Zeitspanne wird auf der Meßuhr der Weg abgelesen, den der von einer Gewindespindel der Spritzenpumpe vorgeschobene Spritzenkolben zurückgelegt hat. Diese Wegmessung hat den Nachteil, daß eine präzise Ablesung der Meßwerte sowie die exakte Einhaltung der ausgewählten Zeitspanne schwierig sind, so daß die Förderrate der Spritzenpumpe nicht zuverlässig mit der erforderlichen Genauigkeit ermittelt werden kann. Zudem ist diese indirekte Messung der Förderrate sehr umständlich und zeitaufwendig.

Der vorliegenden Erfindung liegt die Aufgabe zugrunde, ein Prüfgerät anzugeben, mit dem auf einfache Weise die relevanten Parameter aller gängigen Arten von Spritzenpumpen und Infusionspumpen präzise gemessen werden können, und zwar insbesondere die Förderrate, der Förderdruck, die Fördermenge und der Abschaltdruck dieser Pumpen.

Diese Aufgabe wird erfindungsgemäß durch die im Kennzeichen des Patentanspruchs 1 angegebenen Merkmale gelöst. Vorteilhafte Weiterbildungen der Erfindung sind in den abhängigen Patentansprüchen gekennzeichnet.

Das erfindungsgemäße Prüfgerät enthält eine Spritze, vorzugsweise eine Glaskolbenspritze, deren Fassungsvermögen zweckmäßigerweise 50 ml betragen kann, einen mit dem Spritzengehäuse verbundenen Anschluß zum Zuführen einer Flüssigkeit von der zu prüfenden Pumpe ins Innere des Spritzengehäuses, wodurch der Spritzenkolben in dem Spritzengehäuse entsprechend zurückgeschoben wird, eine Wegmeßeinrichtung, die mit dem Spritzenkolben mechanisch fest verbunden ist, so daß die Bewegung des Kolbens auf die Wegmeßeinrichtung übertragen wird, eine mit der Wegmeßeinrichtung verbundene Rechner- und Auswerte-Einrichtung sowie eine mit der letzteren verbundene Anzeigeeinrichtung für die Förderrate und/oder die Fördermenge der in das Spritzengehäuse geförderten Flüssigkeit. Diese Bauteile sind zweckmäßigerweise innerhalb eines Gehäuses angeordnet, das zum Anschluß der zu testenden Pumpe einen Luer-Lock-Schlauchanschluß haben kann. Mit diesem Luer-Lock-Anschluß wird die zu prüfende Pumpe mittels eines wassergefüllten Schlauches konnektiert, woraufhin bei der Pumpe die gewünschte Förderrate eingestellt wird, so daß diese die Flüssigkeit in die Spritzeneinrichtung des Prüfgerätes fördert, nachdem das Prüfgerät eingeschaltet wurde. Da der Kolben der Spritzeneinrichtung mechanisch starr mit der Wegmeßeinrichtung verbunden ist, wird über die Bewegung des Spritzenkolbens die Wegmeßeinrichtung entsprechend betätigt, die zugeordnete elektrische Signale an die Rechner- und Auswerte-Elektronik weitergibt.

Da der Innendurchmesser der Spritze bekannt ist und der zurückgelegte Weg des Kolbens der Spritze durch die Wegmeßeinrichtung ermittelt wird, errechnet die Rechen- und Auswerte-Einrichtung aus diesen Werten die Fördermenge der Flüssigkeit. Die Förderrate ist eine Zeitfunktion dieser Fördermenge. Somit können durch die Anzeigeeinrichtung die jeweiligen Werte der Förderrate und der Fördermenge der Flüssigkeit in entsprechenden Anzeigefeldern angezeigt werden.

Die Ermittlung dieser Werte erfolgt schnell und äußerst präzise, wozu der jeweilige Prüfling lediglich über einen wassergefüllten Schlauch an das Prüfgerät angeschlossen werden muß. Die Prüfung kann daher auch von Nicht-Fachleuten ausgeführt werden.

Außerdem sieht die Erfindung vor, daß eine Gegenkrafteinrichtung dem Vorschub des Spritzenkolbens bei der Zuführung der Flüssigkeit in das Spritzengehäuse entgegenwirkt, wobei diese Gegenkrafteinrichtung den Kolben stets in Richtung seiner Ausgangslage beaufschlagt. Über diese Krafteinrichtung wird der Förderdruck eingestellt.

In einer zweckmäßigen Ausgestaltung der Erfindung ist die Gegenkrafteinrichtung eine Druckfeder, die zwischen dem rückwärtigen Ende des Kolbens bzw. einer mit dem Kolben verbundenen Kolbenstange und einem beispielsweise an einer Gehäusewand des Prüfgerätes angebrachten Federsitz angeordnet sein kann, der mittels einer Einstellschraube verstellbar sein kann.

Mit großem Vorteil wird ferner vorgeschlagen, daß das Prüfgerät ferner eine Druckmeßeinrichtung für die dem Spritzengehäuse zugeführte Flüssigkeit aufweist. Diese Druckmeßeinrichtung sollte ein Druck-Meß-Wandler sein, dessen Meßdaten von einer zugehörigen Förderdruckanzeigeeinrichtung des Gerätes angezeigt werden. Hierbei kann die Anordnung so getroffen sein, daß der Druck-Meß-Wandler über ein T-förmiges Leitungsstück mit dem Spritzeneinlaß verbunden ist.

Die Wegmeßeinrichtung ist zweckmäßigerweise ein Linear-Potentiometer, mit dessen Spindel der Spritzenkolben starr verbunden sein sollte. Die Anordnung wird innerhalb des Gerätegehäuses zweckmäßigerweise so getroffen, daß das Linear-Potentiometer im Abstand neben der Spritzeneinrichtung angeordnet ist, wobei die Längsachsen der beiden Bauteile parallel zueinander verlaufen. Der Spritzenkolben ist zweckmäßigerweise über einen starren Verbindungsarm mit der Spindel des Potentiometers gekoppelt. Auf diese Weise wird jede Bewegung des Spritzenkolbens exakt auf das Linear-Potentiometer übertragen.

Es liegt im Rahmen der Erfindung, daß anstelle eines Linear-Potentiometers andere Arten linearer Wegmeßeinrichtungen verwendet werden.

Mit großem Vorteil wird ferner vorgeschlagen, daß das Gerät einen festen Anschlag aufweist, der den Hub des Spritzenkolbens begrenzt. Wenn der Spritzenkolben am Schluß eines Meßvorganges auf diesen gehäusefesten Anschlag aufläuft, steigt der Förderdruck an, woraufhin die zu prüfende Pumpe in den Alarmzustand schaltet. Es ist ferner erfindungsgemäß vorgesehen, daß das Prüfgerät einen Alarmeingang hat, mit dem der Alarmausgang der zu prüfenden Pumpe elektrisch verbindbar ist. Auf diese Weise wird erfindungsgemäß das Alarmsignal des Prüflings elektrisch ausgewertet und dazu benutzt, die drei Anzeigen: Förderdruck, Förderrate und Fördermenge elektronisch einzuspeichern. Anschließend wird die zu prüfende Pumpe diskonnektiert, woraufhin die Druckfeder den Spritzenkolben wieder in die Ausgangslage zurückdrückt.

Als Spritzeneinrichtung wird vorteilhafterweise eine Glaskolbenspritze verwendet, die -wie bereits oben erwähntvorzugsweise ein Fassungsvermögen von 50 ml hat. Die Glaskolbenspritze steht zweckmäßigerweise mit einem Luer-Lock-Anschluß in Verbindung, an dem ein wassergefüllter Schlauch angeschlossen wird.

Wesentlich ist, daß nicht nur der angeschlossene Schlauch blasenfrei mit Wasser gefüllt ist, sondern auch der anschließende Spritzeneinlaß bis zum Spritzenkolben, so daß sichin diesem gesamten Leitungssystem keine (komprimierbare) Luft befindet. DIes kann auf einfache Weise dadurch ermöglicht werden, daß (entgegen der rein schematischen Figur) die Glaskolbenspritze mit dem Spritzeneinlaß vertikal (oder schräg) in dem Gerät angeordnet ist, so daß beim Einfüllen von Wasser in den Spritzeneinlaß sämtliche Luft entweicht. Dies kann selbstverständlich auch durch ein oder mehrere Entlüftungsventile oder dergleichen erreicht werden.

Bei Spritzen- und Infusionspumpen hängt die Förderrate im beträchtlichen Maße von dem entgegenwirkenden Druck ab. Um die Förderrate in dem der Pumpe zugeordneten Arbeits-Druckbereich messen zu können, stellt die das Prüfgerät handhabende Bedienungsperson zu Beginn eines Prüfvorganges mit einem Blick auf die Anzeigeeinrichtung fest, wie groß der Anfangsförderdruck ist. Dieser Anfangsförderdruck wird erforderlichenfalls z.B. durch Verstellen des Federsitzes auf den zur Pumpe gehörenden korrekten Druckwert eingestellt. Dies ist schnell und einfach zu bewerkstelligen und stellt sicher, daß die Förderrate in dem angegebenen Arbeitsdruckbereich der Pumpe gemessen wird.

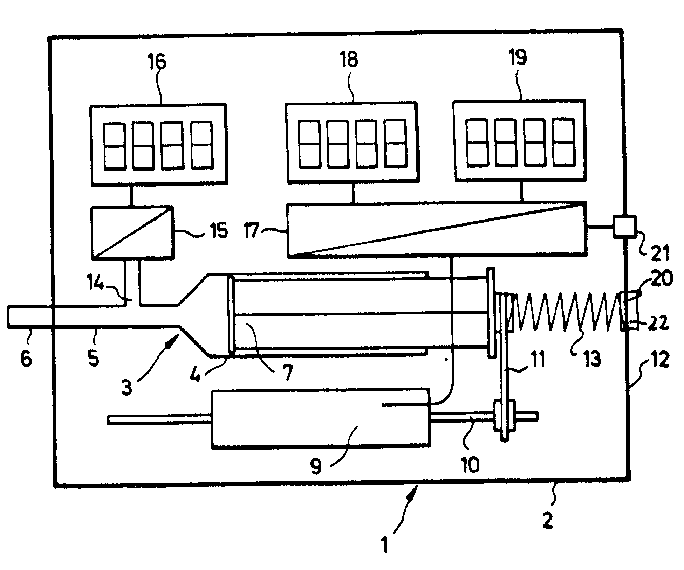

Weitere Merkmale, Vorteile und E inzelheiten der Erfindung ergeben sich aus der nachfolgenden Beschreibung einer bevorzugten Ausführungsform der Erfindung sowie anhand der Zeichnung. Dabei zeigt die einzige Figur auf rein schematische Weise eine Ausführungsform des erfindungsgemäßen Prüfgerätes.

Das in der rein schematischen Figur allgemein mit 1 bezeichnete Prüfgerät bzw. Meßgerät enthält in einem Kunststoffgehäuse 2 eine Glaskolbenspritze 3, in deren Zylinder 4 ein Spritzeneinlaß 5 einmündet, der mit einem ebenfalls nur schematisch angedeuteten Luer-Lock-Anschluß 6 verbunden ist.

In dem Spritzengehäuse 4 ist ein Kolben 7 verschieblich geführt, der einen Wasseraufnahmeraum 8 in dem Zylinder 4 begrenzt.

Parallel zu der Glaskolbenspritze 3 ist -neben dieser liegendein Linear-Potentiometer 9 angeordnet, dessen Spindel 10 über einen Verbindungsarm 11 starr mit dem rückwärtigenEnde des Kolbens 7 verbundenist. Damit wird eine axiale Bewegung des Kolbens 7 in eine parallele Bewegung der Spindel 10 des Linear-Potentiometers 9 übertragen.

Zwischen dem rückwärtigen Ende des Kolbens 7 und der Wand 12 des Gehäuses 2 ist eine Spiral-Druckfeder 13 angeordnet, die den Kolben 7 in seine in der Figur linke Ausgangsstellung beaufschlagt und einer Bewegung des Kolbens 7 nach rechts entgegenwirkt. Über die Feder 13 wird damit der Förderdruck der zugeführten Flüssigkeit (Wasser) in den Innenraum 8 eingestellt.

Mit dem Spritzeneinlaß 5 ist über T-Stück 14 ein Druck-Meß-Wandler 15 verbunden. Dieser Druck-Meßverstärker steht mit einer zugeordneten Anzeige 16 in Verbindung, die mit einer 7-Segment-LD-Anzeige den jeweiligen Förderdruck an der Oberseite des Gehäuses 2 anzeigt.

Das Linear-Potentiometer 9 ist mit einer Rechner- und Auswerte-Elektronik 17 verbunden, dem das Linear-Potentiometer 9 die der Bewegung des Kolbens 7 entsprechende Ausgangswerte zuführt. Die jeweils bereits geförderte Menge sowie die jeweilige Förderrate werden auf den angeschlossenen Anzeigen 18 und 19 angezeigt.

Um die relevanten Parameter einer Spritzenpumpe oder einer Infusionspumpe zu ermitteln bzw. zu überprüfen, wird der Prüfling mittels eines wassergefüllten Schlauches an den Luer-Lock-Anschluß 6 konnektiert. Nach Einstellung der gewünschten Förderrate an der Pumpe drückt diese die Flüssigkeit in den Innenraum 8 der Glaskolbenspritze 3, wodurch der Kolben 7 in der Figur nach rechts gedrückt wird. Dabei wird über den Druck-Meß-Wandler 15 auf dem Anzeigefeld 16 der jeweilige Förderdruck angezeigt.

Da der Glaskolben 7 der Spritze 3 mechanisch starr mit der Spindel 10 des Linear-Potentiometers verbunden ist und das letztere die seiner Bewegung entsprechenden elektrischen Signale an die Rechen- und Auswerte-Elektronik 17 weitergibt, werden auf den Anzeigefeldern 18 und 19 fortlaufend die Förderrate und die jeweilige Fördermenge angezeigt. Da der Innendurchmesser der Glaskolbenspritze 3 bekannt ist, lassen sich diese Werte über den zurückgelegten Wert des Kolbens 7 auf einfache Weise errechnen.

Am Ende des Meßvorganges läuft das rückwärtige Ende des Kolbens 7 direkt oder unter Zwischenschaltung eines weiteren Bauteils auf einen gehäusefesten Anschlag 20 auf, so daß der Kolben nicht weiter verschoben werden kann. Dies hat zur Folge, daß der Förderdruck ansteigt und die zu prüfende Pumpe in den Alarmzustand schaltet. Der Alarmausgang des Prüflings ist elektrisch mit einem Alarmeingang 21 des Prüfgerätes 1 verbunden. Das nun empfangene Alarmsignal des Prüflings wird elektrisch ausgewertet und dazu benutzt, die drei Anzeigen Abschaltdruck der Pumpe, Förderrate und Fördermenge elektronisch einzuspeichern. Damit ist der Prüfvorgang beendet, wobei nach Lösen des Anschlußschlauches von dem Luer-Lock-Anschluß 6 die Feder 13 wieder den Kolben 7 in die Ausgangsposition zurückdrückt.

Die Spiral-Druckfeder 13 wird so eingestellt, daß dem Vorschub des Kolbens 7 ein geeigneter Anfangsdruck entgegenwirkt, wobei der Federdruck im Verlaufe der Vorschubbewegung des des Kolbens exponentiell ansteigt.

Hierzu ist der Anschlag 20 als verstellbarer Federsitz ausgebildet, der mittels einer außen am Gehäuse 2 angeordneten Rändelschraube 22 einstellbar ist.

In einer alternativen Ausgestaltung der Erfindung kann vorgesehen sein, daß anstelle der Spiral-Druckfeder 13 eine mechanische Reibungsbremse angeordnet ist, die an der Spindel 10 des Linear-Potentiometers 9 oder auch direkt an dem Kolben 7 angreifen kann, wobei diese Reibungsbremse im Gegensatz zu der Spiral-Druckfeder 13 dem Vorschub des Kolbens mit einer über den Weg konstant bleibenden Kraft entgegenwirkt. Die Reibungskraft dieser Reibungsbremse ist z.B. mittels eines Klemm-Mechanismus einstellbar.

Das Prüfgerät ist auch auf andere Arten von Pumpen anwendbar.

## Patentansprüche

1. Prüfgerät für Spritzen- und Infusionspumpen, mit einem Gehäuse, in dem ein Kolben verschieblich geführt ist, der den Innenraum des Gehäuses begrenzt, einer Wegmeßeinrichtung, die mit dem Kolben verbunden ist, einem mit dem Gehäuse verbundenen Anschluß für eine mit der Pumpe verbundene Leitung zum Zuführen einer Flüssigkeit in das Gehäuse, einer mit der Wegmeßeinrichtung verbundene Rechner- und Auswerteeinrichtung, einer mit der Rechner- und Auswerteeinrichtung verbundene Anzeigeeinrichtung für die Förderrate und/oder Fördermenge der zugeführten Flüssigkeit und mit einer Einrichtung, die eine dem Vorschub des Kolbens entgegenwirkende Kraft ausübt,
**dadurch gekennzeichnet,**
daß die Kraft der Gegenkrafteinrichtung (13) einstellbar ist.

2. Prüfgerät nach Anspruch 1,
dadurch gekennzeichnet, daß eine Druckmeßeinrichtung (15) den Druck der Flüssigkeit in dem Gehäuse (4) erfaßt.

3. Prüfgerät nach Anspruch 1 oder 2,
dadurch gekennzeichnet, daß die Gegenkrafteinrichtung eine Druckfeder (13) ist, die zwischen dem rückwärtigen Ende des Kolbens (7) oder einer mit demKolben verbundene Kolbenstange und einer Gehäusewand (12) des Prüfgerätes angeordnet ist.

4. Prüfgerät nach Anspruch 3,
dadurch gekennzeichnet, daß der Federsitz der Druckfeder (13) mittels einer Einstellschraube verstellbar ist.

5. Prüfgerät nach Anspruch 1 oder 2,
dadurch gekennzeichnet, daß die Gegenkrafteinrichtung eine mechanische Reibungsbremse ist, deren Reibungskraft einstellbar ist.

6. Prüfgerät nach einem der Ansprüche 1 bis 5,
dadurch gekennzeichnet, daß die Druckmeßeinrichtung ein Druck-Meß-Wandler (15) ist, dessen Meßdaten von einer Förderdruckanzeigeeinrichtung (16) anzeigbar sind.

7. Prüfgerät nach einem der Ansprüche 1 bis 6,
dadurch gekennzeichnet, daß der Flüssigkeitszufuhr-Anschluß ein Luer-Lock-Anschluß (6) ist, an den ein mit Flüssigkeit gefüllter, mit dem zu prüfenden Gerät verbundener Schlauch anschließbar ist.

8. Prüfgerät nach einem der Ansprüche 1 bis 7, gekennzeichnet durch einen Alarmeingang (21), der mit dem Alarmausgang der zu prüfenden Pumpe elektrisch verbindbar ist.

9. Prüfgerät nach einem der Ansprüche 1 bis 8, ferner gekennzeichnet durch ein Kunststoffgehäuse (2), das seitlich mit dem Luer-Lock-Anschluß, der Alarmeingangsbuchse (21) und einem Netzanschluß versehen ist und an der Oberseite drei Anzeigefelder (16, 18, 19) für den Förderdruck, die Förderrate und die Fördermenge aufweist.

10. Prüfgerät nach einem der Ansprüche 1 bis 9, dadurch gekennzeichnet, daß der gemessene Abschaltdruck gespeichert und angezeigt wird.

Europäisches
Patentamt

# EUROPÄISCHER RECHERCHENBERICHT

Nummer der Anmeldung

EP 91 12 1823

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,P | DE-C-4 000 873 (A.S.M GMBH) <br> * Zusammenfassung * <br> --- | 1 | A61M5/145 |
| A | EP-A-0 285 679 (B.BRAUN-SSC AG) <br> * Spalte 4, Zeile 28 - Zeile 52; Abbildung * <br> --- | 1 | |
| A | FR-A-2 365 349 (PYE LTD) <br> * Anspruch 3; Abbildungen * <br> --- | 1 | |
| A | GB-A-2 116 703 (WAUGH CONTROLS) <br> * Zusammenfassung; Abbildung 1 * <br> ----- | 1 | |

**RECHERCHIERTE SACHGEBIETE (Int. Cl.5 )**

A61M
F04B
G01F

Der vorliegende Recherchenbericht wurde für alle Patentansprüche erstellt

| Recherchenort | Abschlußdatum der Recherche | Prüfer |
|---|---|---|
| DEN HAAG | 25 MAERZ 1992 | CLARKSON P. |